# EUROPÄISCHE PATENTSCHRIFT

(11) **EP 0 619 302 B1**
(45) Veröffentlichungstag und Bekanntmachung des Hinweises auf die Patenterteilung: **12.03.1997**
(21) Anmeldenummer: 94105023.9
(22) Anmeldetag: 30.03.1994
(51) Int. Cl.: C07C 309/40, C07C 303/06

(54) **Verfahren zur Herstellung von kristallisierten, salzfreien chlorsubstituierten 3-Nitrobenzolsulfonsäure-Hydraten**
Process for the preparation of crystallized, salt-free, chlorosubstituted 3-nitrobenzene sulfonic acid hydrates
Procédé pour la préparation de hydrates cristallisés et dépourvus de sels d'acides 3-nitrobenzène sulfonique chlorosubstitués

(30) Priorität: 06.04.1993 DE 4311381
(43) Veröffentlichungstag der Anmeldung: 12.10.1994
(73) Patentinhaber: HOECHST AKTIENGESELLSCHAFT, 65926 Frankfurt am Main (DE)
(72) Erfinder: Arndt, Otto, Dr., D-65719 Hofheim/Ts. (DE)

(56) Entgegenhaltungen:
- DD-A- 86 391
- DE-A- 2 621 168
- DE-A- 3 501 754

## Beschreibung

Die Erfindung betrifft ein Verfahren zur Herstellung von kristallisierten, salzfreien, chlorsubstituierten 3-Nitrobenzolsulfonsäure-Hydraten aus Sulfonierungsgemischen, die chlorsubstituierte 3-Nitrobenzolsulfonsäure enthalten.

Die Herstellung der chlorsubstituierten 3-Nitrobenzolsulfonsäuren ist in der Literatur beschrieben (z.B. Ullmann, Encyclopädie der technischen Chemie 4. Auflage 1979, Band 17, S. 405-408). Bekannt sind diskontinuierliche und kontinuierliche Verfahren einerseits zur Nitrierung der entsprechenden Benzolsulfonsäuren und andererseits zur Sulfonierung der entsprechenden Nitrobenzole. Wegen des geringeren Sicherheitsrisikos werden die Sulfonierungsverfahren den Nitrierverfahren vorgezogen.

Die entsprechenden 3-Nitrobenzolsulfonsäuren können durch Vermischen der Reaktionsgemische mit Wasser und Natriumhydroxid bzw. Calciumhydroxid und NaCl in Form ihrer Natriumsalze isoliert werden. Dies ist problematisch, da hierbei die Bildung großer Mengen Natriumsulfat und Calciumsulfat zu einer hohen Salzbelastung des Abwassers führt. Deshalb ist die Isolierung als freie Säure vorteilhafter. Die Reaktionsgemische werden hierzu mit Wasser bzw. - zwecks Herabsetzung der Mischwärme - mit wasserhaltiger Schwefelsäure vereinigt, wobei die freien Sulfonsäuren auskristallisieren. Sie werden durch Filtration isoliert.

In der DDR Patentschrift 86 391 ist ein derartiges Verfahren zur kontinuierlichen Gewinnung von 4-Nitrotoluol-2-sulfonsäure aus einem Sulfonierungsgemisch, dem 40 bis 50%ige wäßrige Schwefelsäure zugesetzt wird, beschrieben.

Bei dieser auf die Gewinnung von 4-Nitrotoluol-2-sulfonsäure beschränkten Arbeitsweise wird allerdings ein Produkt erhalten, dem eine große Menge Schwefelsäure anhaftet.

Hinsichtlich der Zielsetzung, nicht nur ein möglichst reines Produkt zu erhalten sondern auch einen möglichst großen Anteil der Abfallschwefelsäure einer Wiederverwertung und/oder einem Wiedereinsatz zuzuführen, läßt das in der DDR Patentschrift 86 391 beschriebene Verfahren jedoch Wünsche offen.

Es bestand die Aufgabe, ein der vorstehend erwähnten Zielsetzung entsprechendes Verfahren zu entwickeln, das sich auf chlorsubstituierte 3-Nitrobenzolsulfonsäuren anwenden und sich zudem technisch einfach realisieren läßt.

Diese Aufgabe wird gelöst durch ein Verfahren zur Herstellung von kristallisierten, salzfreien chlorsubstituierten 3-Nitrobenzolsulfonsäure-Hydraten aus chlorsubstituierte 3-Nitrobenzolsulfonsäure enthaltenden Sulfonierungsgemischen. Es ist dadurch gekennzeichnet, daß man das Sulfonierungsgemisch mit 8 - 25 Gew.-%iger wäßriger Schwefelsäure bei 40°C bis 100°C vermischt, die anfallende Suspension auf 5°C bis 20°C abkühlt, den Niederschlag abfiltriert, den Niederschlag mit Wasser bei 30°C bis 100°C auflöst, gegebenenfalls filtriert, die Lösung auf 5 bis 40°C, vorzugsweise auf 10° bis 40°C abkühlt, filtriert und das Schwefelsäure enthaltende Filtrat gegebenenfalls rezykliert.
Angesichts der guten Löslichkeit der Sulfonsäuren in Wasser ist hierbei besonders überraschend, daß der durch den zusätzlichen Verfahrensschritt bedingte Ausbeuteverlust nur minimal ist.
Durch das erfindungsgemäße Verfahren erhält man zum einen chlorsubstituierte 3-Nitrobenzolsulfonsäure-Hydrate in sehr reiner Form und gewinnt zum anderen einen sehr hohen Teil wiederverwertbarer Schwefelsäure.
Aus den Sulfonsäure-Hydraten lassen sich ohne großen Aufwand entweder die freien Sulfonsäure oder Salze der Sulfonsäuren herstellen.
Das Sulfonierungsgemisch enthält, bedingt durch seine Herstellung, üblicherweise konzentrierte Schwefelsäure und/oder Oleum. Durch die Zugabe von verdünnter wäßriger Schwefelsäure wird die konzentrierte Schwefelsäure verdünnt und das Oleum in Schwefelsäure umgewandelt. Hierbei fällt die chlorsubstituerte 3-Nitrobenzolsulfonsäure in Form eines Hydrates aus und wird abfiltriert.
Der hierbei anfallende Anteil Schwefelsäure eignet sich aufgrund seines Schwefelsäuregehaltes (etwa von 40 bis 50 Gew.% Schwefelsäure) in besonderem Maße für eine Regenerierung. Das aus dem Sulfonierungsgemisch durch Zusatz verdünnter wäßriger Schwefelsäure und nachfolgender Kühlung der Suspension ausgefällte Hydrat der chlorsubstituierten 3-Nitrobenzolsulfonsäure wird abfiltriert und anschließend in warmem Wasser aufgelöst. Diese wäßrige Lösung wird zweckmäßigerweise in zwei Schritten gekühlt und vom ausgefallenen chlorsubstituierten 3-Nitrobenzolsulfonsäure-Hydrat abfiltriert. Das beim Abfiltrieren des Hydrates anfallende, Schwefelsäure enthaltende Filtrat kann besonders vorteilhaft zur Vermischung mit dem Sulfonierungsgemisch verwendet werden und die dabei anfallende Schwefelsäure wiederum einer Regenerierung zugeführt werden. Auf diese Weise läßt sich die anfallende Schwefelsäure in einem sehr hohem Maße sinnvoll nutzen und die Bildung von unerwünschter, nicht wiederverwertbarer Abfallschwefelsäure weitestgehend vermeiden.
Das Verfahren eignet sich für ein Sulfonierungsgemisch, das als chlorsubstituierte 3-Nitrobenzolsulfonsäure 6-Chlor-3-nitrobenzolsulfonsäure oder 4-Chlor-3-nitrobenzolsulfonsäure, insbesondere 6-Chlor-3-nitrobenzolsulfonsäure, enthält.

In vielen Fällen empfiehlt es sich, das Sulfonierungsgemisch mit 10 bis 20 Gew.-%iger, insbesondere 10 bis 15 Gew.-%iger wäßriger Schwefelsäure bei einer Temperatur von 40°C bis 75°C, insbesondere 45°C bis 65°C, vorzugsweise 50°C bis 60°C zu vermischen.
Der größte Teil der bei der Fällung freiwerdenden Wärme wird von der Verdünnungsschwefelsäure aufgenommen. Die Kühlung läßt sich deshalb einfach steuern.

Es hat sich häufig bewährt, die anfallende Suspension auf 10 bis 18°C, vorzugsweise 12 bis 16°C abzukühlen, den erhaltenen Niederschlag in Wasser bei 45°C bis 80°C, vorzugsweise 50 bis 60°C aufzulösen und die erhaltene Lösung zunächst auf 25 bis 35°C und anschließend auf 5 bis 15°C abzukühlen. Das Verfahren läßt sich diskontinuierlich und auch kontinuierlich durchführen.

Das nachstehende Beispiel dient zur Erläuterung des erfindungsgemäßen Verfahrens, ohne es darauf zu beschränken.

### Beispiel 1

### 1a) Herstellung des rohen Dihydrates (1. Fällung)

158,4 Gew.-Teile eines durch Umsetzung von 63,0 Gewichtsteilen 4-Chlornitrobenzol mit 86,3 Gewichtsteilen 65 %igem Oleum (= 65 Teile SO₃ und 35 Teile H₂SO₄) und 9,1 Gewichtsteilen H₂SO₄ erhaltenen Sulfonierungsgemisches (Dichte von 1,64 g/ml) wird in einem emaillierten, mit Ankerrührer und Kühlmöglichkeiten ausgestatteten Verdünnungs- und Fällungsgefäß mit 180,0 Gewichtsteilen einer 11 %igen Schwefelsäure (bzw. verdünnter Filtratschwefelsäure aus der 2. Fällung) unter Rühren mit hoher Tourenzahl und mäßiger Außenkühlung bei 50 - 60°C vermischt. Nach einer Verweilzeit von ca. 2 Stunden bei 50 - 60°C unter Rühren läuft die dünne Suspension in ein emailliertes, mit Ankerrührer und Kühlmöglichkeiten ausgestattetes Nachrührgefäß ab, wo nach einer Verweilzeit von mindestens 5 Stunden bei 15°C die Kristallisation ebenfalls unter Rühren vervollständigt wird. Die dünne Suspension mit einem Feststoffgehalt von 31 % wird abfiltriert. Man erhält ca. 163,0 Gewichtsteile 6-Chlor-3-nitrobenzolsulfonsäure-Dihydrat (technisch feucht) mit einem Gehalt von 103,0 Gewichtsteilen 6-Chlor-3-nitrobenzolsulfonsäure und 21,0 Gewichtsteilen H₂SO₄ (je 100 %ig gerechnet).

Das Filtrat (150,8 Gewichtsteile mit einer Dichte von 1,36) ist eine 44 %ige Schwefelsäure mit einem Gehalt von 66,6 Gewichtsteilen H₂SO₄ (100 %ig gerechnet).

Aus dem Filtrat werden 1,5 Gewichtsteile als Rückführschwefelsäure entnommen und mit Filtratschwefelsäure aus der 2. Fällung vereinigt.

Die Hauptmenge des Filtrats (149,3 Gewichtsteile mit einem Gehalt von 65,8 Gewichtsteilen H₂SO₄ (100 %ig gerechnet, entsprechend 96 % der gesamten Abfallschwefelsäure) gehen zur Regeneration.

Das 6-Chlor-3-nitrobenzolsulfonsäure-Dihydrat (technisch feucht) geht unmittelbar in das emaillierte mit Ankerrührer und Kühlmöglichkeit ausgestattete Gefäß für die 2. Fällung.

### 1b) 2. Fällung

Man legt 80,0 Gewichtsteile 50°C warmes Wasser vor und trägt 163,0 Gewichtsteile 6-Chlor-3-nitrobenzolsulfonsäure-Dihydrat technisch feucht unter Rühren ein. Man erwärmt dabei auf 50°C. Bis auf das als Feststoff zurückbleibende Sulfon geht alles in Lösung. Man läßt zur Kristallisation unter langsamem Rühren zunächst auf ca. 30°C, dann, unter Verwendung von Kühlsole, auf 10°C abkühlen. Man rührt 1 Stunde nach. Die gut rührbare Suspension wird bei 10°C abfiltriert.

Man erhält 120,0 Gewichtsteile 6-Chlor-3-nitrobenzolsulfonsäure-Dihydrat (technisch feucht) mit einem Gehalt von 106,0 Gewichtsteilen 6-Chlor-3-nitrobenzolsulfonsäure-Dihydrat (= 92,0 Gewichtsteilen 6-Chlor-3-nitrobenzolsulfonsäure), 3,0 Gewichtsteilen H₂SO₄ je 100 %ig gerechnet und 25,0 Gewichtsteilen Wasser.

Die ablaufende Filtratschwefelsäure (= 115,0 Gewichtsteile) enthält 10,4 Gewichtsteile 6-Chlor-3-nitrobenzolsulfonsäure und 19,0 Gewichtsteile H₂SO₄, je 100 %ig gerechnet.
Man korrigiert auf einen Gehalt von 19,6 Gewichtsteile H₂SO₄ 100 %ig. Die Ergänzungsmenge an Schwefelsäure richtet sich nach dem Schwefelsäuregehalt der aus der Umlösung resultierenden Filtratschwefelsäure.

Man verdünnt die Filtratschwefelsäure mit ca. 62,5 Gewichtsteilen Wasser auf eine Menge von 180,0 Gewichtsteile (Dichte = 1,10 g/ml Gehalt H₂SO₄ = 10,9 Gew.%).

Die so erhaltene Säure wird zur Vermischung mit weiterem Sulfonierungsgemisch und Fällung weiteren 6-Chlor-3-nitrobenzolsulfonsäure-Dihydrates eingesetzt.

## Patentansprüche

1. Verfahren zur Herstellung von kristallisierten, salzfreien chlorsubstituierten 3-Nitrobenzolsulfonsäure-Hydraten aus chlorsubstituierte 3-Nitrobenzolsulfonsäure enthaltenden Sulfonierungsgemischen, dadurch gekennzeichnet, daß man das Sulfonierungsgemisch mit 8 bis 25 Gew.-%iger, wäßriger Schwefelsäure bei 40 bis 100°C vermischt, die anfallende Suspension auf 5 bis 20°C abkühlt, den Niederschlag abfiltriert, den Niederschlag mit Wasser bei 30° bis 100°C auflöst, gegebenenfalls filtriert, die Lösung auf 5° bis 40°C abkühlt, filtriert und das Schwefelsäure enthaltende Filtrat gegebenenfalls rezykliert.

2. Verfahren nach Anspruch 1,.dadurch gekennzeichnet, daß das Sulfonierungsgemisch als chlorsubstituierte 3-Nitrobenzolsulfonsäure 6-Chlor-3-nitrobenzolsulfonsäure 6-Chlor-3-nitrobenzolsulfonsäure oder 4-Chlor-3-nitrobenzolsulfonsäure, insbesondere enthält.

3. Verfahren nach einem oder mehreren der Ansprüche 1 bis 2, dadurch gekennzeichnet, daß man das Sulfonierungsgemisch mit 10 bis 20 Gew.-%iger, insbesondere 10 bis 15 Gew.-%iger wäßriger Schwefelsäure vermischt.

4. Verfahren nach einem oder mehreren der Ansprüche 1 bis 3, dadurch gekennzeichnet, daß man das Sulfonierungsgemisch mit wäßriger Schwefelsäure bei 45 bis 65°C, insbesondere 50 bis 60°C vermischt.

5. Verfahren nach einem oder mehreren der Ansprüche 1 bis 4, dadurch gekennzeichnet, daß man die anfallende Suspension auf 10 bis 18°C, insbesondere 12 bis 16°C abkühlt.

6. Verfahren nach einem oder mehreren der Ansprüche 1 bis 5, dadurch gekennzeichnet, daß man den Niederschlag mit Wasser bei 50 bis 60°C auflöst.

7. Verfahren nach einem oder mehreren der Ansprüche 1 bis 6, dadurch gekennzeichnet, daß man die Lösung zunächst auf 25 bis 35°C und anschließend auf 5 bis 15°C abkühlt.

8. Verfahren nach einem oder mehreren der Ansprüche 1 bis 7, dadurch gekennzeichnet, daß man das Schwefelsäure enthaltende Filtrat mit dem Sulfonierungsgemisch vermischt.

## Claims

1. A process for the preparation of crystalline, salt-free, chlorine-substituted 3-nitrobenzenesulfonic acid hydrates from sulfonation mixtures which contain chlorine-substituted 3-nitrobenzenesulfonic acid, which comprises mixing the sulfonation mixture with from 8 to 25% strength by weight aqueous sulfuric acid at from 40 to 100°C, cooling the resulting suspension to from 5 to 20°C, filtering off the precipitate and dissolving it with water at from 30 to 100°C, carrying out filtration if desired, cooling the solution to from 5 to 40°C and filtering it, and recycling the sulfuric acid-containing filtrate if desired.

2. The process as claimed in claim 1, wherein the sulfonation mixture contains 6-chloro-3-nitrobenzenesulfonic acid or 4-chloro-3-nitrobenzenesulfonic acid, in particular 6-chloro-3-nitrobenzenesulfonic acid, as chlorine-substituted 3-nitrobenzenesulfonic acid.

3. The process as claimed in either or both of claims 1 and 2, wherein the sulfonation mixture is mixed with from 10 to 20% strength by weight, in particular from 10 to 15% strength by weight, aqueous sulfuric acid.

4. The process as claimed in one or more of claims 1 to 3, wherein the sulfonation mixture is mixed with aqueous sulfuric acid at from 45 to 65°C, in particular from 50 to 60°C.

5. The process as claimed in one or more of claims 1 to 4, wherein the suspension which is obtained is cooled to from 10 to 18°C, in particular from 12 to 16°C.

6. The process as claimed in one or more of claims 1 to 5, wherein the precipitate is dissolved with water at from 50 to 60°C.

7. The process as claimed in one or more of claims 1 to 6, wherein the solution is cooled initially to from 25 to 35°C and subsequently to from 5 to 15°C.

8. The process as claimed in one or more of claims 1 to 7, wherein the sulfuric acid-containing filtrate is mixed with the sulfonation mixture.

## Revendications

1. Procédé de préparation d'hydrates d'acides 3-nitrobenzènesulfoniques dépourvus de sels et cristallisés, à partir de mélanges de sulfonation contenant des acides 3-nitrobenzènesulfoniques chlorosubstitués, caractérisé en ce que l'on mélange, à 40°C jusqu'à 100°C, le mélange de sulfonation avec de l'acide sulfurique aqueux à 8 à 25% en poids, on refroidit la suspension obtenue à 5 jusqu'à 20°C, on sépare le précipité par filtration, on dissout le précipité avec de l'eau à 30° jusqu'à 100°C, on filtre éventuellement, on refroidit la solution à 5° jusqu'à 40°C, on filtre et on recycle éventuellement le filtrat contenant de l'acide sulfurique.

2. Procédé selon la revendication 1, caractérisé en ce que le mélange de sulfonation contient, en tant qu'acide 3-nitrobenzènesulfonique chlorosubstitué, de l'acide 6-chloro-3-nitrobenzènesulfonique ou de l'acide 4-chloro-3-nitrobenzènesulfonique, en particulier de l'acide 6-chloro-3-nitrobenzènesulfonique.

3. Procédé selon l'une ou plusieurs des revendications 1 à 2, caractérisé en ce qu'on mélange le mélange de sulfonation avec de l'acide sulfurique aqueux à 10 à 20% en poids, en particulier à 10 à 15% en poids.

4. Procédé selon l'une ou plusieurs des revendications 1 à 3, caractérisé en ce qu'on mélange le mélange de sulfonation avec de l'acide sulfurique aqueux à 45 jusqu'à 65°C, en particulier à 50 jusqu'à 60°C.

5. Procédé selon l'une ou plusieurs des revendications 1 à 4, caractérisé en ce qu'on refroidit la suspension formée à 10 jusqu'à 18°C, en particulier 12 jusqu'à 16°C.

6. Procédé selon l'une ou plusieurs des revendications 1 à 5, caractérisé en ce qu'on dissout le précipité avec de l'eau à 50 jusqu'à 60°C.

7. Procédé selon l'une ou plusieurs des revendications 1 à 6, caractérisé en ce qu'on refroidit la solution d'abord à 25 jusqu'à 35°C et ensuite à 5 jusqu'à 15°C.

8. Procédé selon l'une ou plusieurs des revendications 1 à 7, caractérisé en ce qu'on mélange le filtrat contenant de l'acide sulfurique avec le mélange de sulfonation.
